# EUROPEAN PATENT APPLICATION

(11) **EP 3 178 921 A1**
(43) Date of publication of application: **14.06.2017**
(21) Application number: 15830582.1
(22) Date of filing: 06.08.2015
(51) Int. Cl.: C12N 1/20, A01G 7/06, A01N 63/00, A01P 21/00

(54) **PLANT GROWTH-PROMOTING AGENT AND PLANT GROWTH-PROMOTING METHOD**

(30) Priority: 07.08.2014 JP 2014161439
(71) Applicant: Kyoto Prefecture, Kyoto-shi, Kyoto 602-8570 (JP); Meiji Seika Pharma Co., Ltd., Tokyo 104-8002 (JP)
(72) Inventor: TSUDA, Kazuhisa, Kameoka-shi Kyoto 621-0846 (JP); UMEMURA, Kenji, Yokohama-shi Kanagawa 222-8567 (JP)
(74) Representative: Krauns, Christian
(86) International application number: PCT/JP2015/003968
(87) International publication number: WO 2016/021204

(57) **Abstract**

A novel plant growth-promoting agent and a plant growth-promoting method using the same are provided. The plant growth-promoting agent contains a lactic acid bacterium having a plant growth-promoting action. Also, in the plant growth-promoting method, a seed, a plant body and/or a soil is treated with a lactic acid bacterium having a plant growth-promoting action. *Lactobacillus plantarum* strain FERM BP-21501 is suitable as the lactic acid bacterium having a plant growth-promoting action.

## Description

### Technical Field

The present invention relates to a plant growth-promoting agent and a plant growth-promoting method which can promote plant growth.

### Background Art

Promoting plant growth and increasing the yields of vegetables, grains, fruits and the like are preferable from the viewpoints of increase in food production and efficient farming. Therefore, various plant growth-promoting agents have been hitherto developed, and plant growth-promoting agents using microorganisms have been also proposed.

For example, PTL 1 discloses that an enhanced fertilizer containing a fertilizer particle, a lactic acid bacterium and a bacterium of Bacillaceae has an effect of enhancing plant growth, development or yield. PTL 2 discloses that a soil improvement material obtained by mixing a water absorbing material produced through graft polymerization of peat and acrylonitrile and a microorganism material produced by adding a microbe such as a lactic acid bacterium to a base material with peat has an effect of promoting the growth of a plant. PTL 3 discloses a plant-based compost containing a fermented material obtained by mixing and fermenting a corn stalk residue, rice bran and bean curd lees, charcoal powder grains and effective microorganisms including a lactic acid bacterium, a yeast fungus and the like. However, these documents do not disclose that lactic acid bacteria themselves have a plant growth-promoting action.

PTL 4 discloses a fertilizer obtained by fermenting a liquid waste discharged during the production of manure using a yeast and a vegetable lactic acid bacterium. In this document, it is disclosed that the fertilizer activates good microorganisms that exercise a favorable influence on the soil due to the functions of the vegetable lactic acid bacterium because the fertilizer contains the three main macronutrients and minerals and further contains the vegetable lactic acid bacterium, while a plant grows without the help of chemical fertilizers or pesticides because diseases and the growth of injurious insects are prevented. However, this document does not disclose that lactic acid bacteria themselves have an action of promoting plant growth, either.

PTL 5 discloses that a plant growth-promoting activity is given to a lactic acid bacterium by mixing and culturing a *Pseudomonas* bacterium having a plant growth-promoting activity with the lactic acid bacterium. However, this document discloses that the original lactic acid bacterium itself does not have a plant growth-promoting action (paragraph 0005), and thus this document does not teach the invention.

PTL 6 discloses that a seed-containing tablet obtained by coating a seed with an effective microbe such as a lactic acid bacterium and its substrate and further coating the coated seed with calcium peroxide and sand secures healthy growth of the seed and a satisfactory yield. In this document, however, a substrate such as chitin and calcium peroxide are essential in addition to the effective microbe, and this document does not disclose the treatment with a lactic acid bacterium alone. Also, a seed is treated with the effective microbe in this document, but this document does not disclose that a growing plant body or a soil is treated with a lactic acid bacterium or that a plant growth-promoting effect is thereby obtained.

### Citation List

### Patent Literature

PTL 1: JP-T-2008-537531 (the term "JP-T" as used herein means a published Japanese translation of a PCT patent application)
PTL 2: JP-A-2007-138123
PTL 3: JP-A-2007-169096
PTL 4: JP-A-2009-007229
PTL 5: JP-A-2009-249301
PTL 6: JP-B-H01-42641
PTL 7: JP-A-2009-201459

### Summary of Invention

### Technical Problem

The present inventors have previously disclosed *Lactobacillus plantarum* strain FERM P-21501 (strain SOK04BY) as a microorganism having a capability of controlling a plant disease in JP-A-2009-201459. On further investigation using strain SOK04BY, the inventors have found that the strain has an action of promoting plant growth and increasing the yield.

This embodiment has been made based on the findings, and an object is to provide a novel plant growth-promoting agent and a plant growth-promoting method using the same.

### Solution to Problem

As this embodiment, the following embodiments are included.
[1] A plant growth-promoting agent containing a lactic acid bacterium having a plant growth-promoting action.
[2] The plant growth-promoting agent according to [1], wherein the lactic acid bacterium having a plant growth-promoting action is a microorganism belonging to *Lactobacillus.*
[3] The plant growth-promoting agent according to [2], wherein the lactic acid bacterium having a plant growth-promoting action is a microorganism classified as *Lactobacillus plantarum.*
[4] The plant growth-promoting agent according to [3], wherein the lactic acid bacterium having a plant growth-promoting action is *Lactobacillus plantarum* strain FERM BP-21501.
[5] The plant growth-promoting agent according to any one of [1] to [4] which is used for treating at least one selected from the group consisting of a seed, a plant body and a soil.
[6] The plant growth-promoting agent according to any one of [1] to [4] which is used for treating a plantlet.
[7] A plant growth-promoting method characterized by treating at least one selected from the group consisting of a seed, a plant body and a soil with a lactic acid bacterium having a plant growth-promoting action.
[8] The plant growth-promoting method according to [7], wherein the lactic acid bacterium having a plant growth-promoting action is *Lactobacillus plantarum* strain FERM BP-21501.
[9] The plant growth-promoting method according to [7] or [8], wherein the plant body that is treated is a plantlet.

### Advantageous Effects of Invention

With the plant growth-promoting agent according to this embodiment, growth of plant body can be promoted, and the yield can be increased, by treating a seed, a plant body or a soil.

### Description of Embodiments

The plant growth-promoting agent according to this embodiment contains a lactic acid bacterium having a plant growth-promoting action.

The lactic acid bacterium used in this embodiment has a capability of promoting plant growth, namely a plant growth-promoting activity. Here, lactic acid bacteria mean those satisfying the conditions of (1) Gram-positive, (2) having rod-shaped or spherical cells, (3) catalase negative, (4) producing lactic acid at 50% or more of glucose consumed, (5) not forming endospores and (6) nonmotile or rarely motile.

Specifically, microorganisms belonging to *Lactobacillus* such as *Lactobacillus plantarum, Lactobacillus mali, Lactobacillus suebicus, Lactobacillus alimentarius, Lactobacillus sakei, Lactobacillus pentosus, Lactobacillus breves, Lactobacillus malefermentans, Lactobacillus lactis, Lactobacillus gasseri, Lactobacillus acidophilus, Lactobacillus bulgaricus* and *Lactobacillus casei*; *Pediococcus* such as *Pediococcus pentosaceus, Pediococcus acidilactici, Pediococcus parvulus*, *Pediococcus damnosus* and *Pediococcus halophilus*; *Lactococcus* such as *Lactococcus lactis*, *Lactococcus raffinolactis* and *Lactococcus plantarum*; *Carnobacterium* such as *Carnobacterium divergens*; *Weissella* such as *Weissella minor*; *Atopobium* such as *Atopobium parvulus*; *Streptococcus* such as *Streptococcus bovis*; *Enterococcus* such as *Enterococcus avium*; *Vagococcus* such as *Vagococcus fluvialis*; *Leuconostoc* such as *Leuconostoc mesenteroides* and *Leuconostoc lactis*; *Oenococcus* such as *Oenococcus oeni*; *Tetragenococcus* such as *Tetragenococcus halophilus* and the like are included. One of these microorganisms may be used alone, or a combination of microorganisms belonging to a same genus or different genera may be used.

Microorganisms belonging to *Lactobacillus* are preferable, and *Lactobacillus plantarum* is more preferable. In particular, *Lactobacillus plantarum* strain FERM BP-21501 (hereinafter referred to as strain SOK04BY) is preferably used.

### Reference to Deposited Biological Material:

Strain SOK04BY is as described in JP-A-2009-201459. Strain SOK04BY has been isolated from salted and fermented squid, and its morphological, cultural and physiological properties are as shown in Table 1 below. Strain SOK04BY has been deposited as follows.

- Depositor: Kyoto Prefectural Agriculture, Forestry and Fisheries Technology Center (An organization of Kyoto Prefecture. Name as of date of national deposition was Kyoto Prefectural Agricultural Resource Research Center. Head of center is Shigetoshi Kitayama. Address is Wakunari 9, Amarubecho, Kameoka-shi, Kyoto 621-0806, Japan.)
- Name of Depositary Authority: International Patent Organism Depositary, National Institute of Technology and Evaluation (Name as of date of national deposition was International Patent Organism Depositary, National Institute of Advanced Industrial Science and Technology.)
- Address of Depositary Authority: Room 120, 2-5-8 Kazusakamatari, Kisarazu-shi, Chiba 292-0818, Japan
- Date of Deposition: February 5, 2008 (date of national deposition)
- Accession No.: FERM BP-21501 (A request for transfer of FERM P-21501, which was deposited in Japan on February 5, 2008, to an international depositary authority under the Budapest Treaty was received on April 30, 2015.)

**[Table 1]**

| Morphological, Cultural and Physiological Properties of Strain SOK04BY | | | | | | | |
|---|---|---|---|---|---|---|---|
| Incubation temperature | | 30°C | | | | Mannose | + |
| Cell morphology | | Bacillus (0.8×1.5-2.0 µm) | | | | Sorbose | - |
| | | | | | | Rhamnose | - |
| Gram's staining | | + | | | | Dulcitol | - |
| Sporulation | | - | | | | Inositol | - |
| Motility | | - | | | | Mannitol | + |
| Colony morphology | | Medium: MRS agar | | | | Sorbitol | + |
| | | Incubation period: 24 hours | | | | α-Methyl-D-mannoside | + |
| | | Diameter: 1.0-2.0 mm | | | | α-Methyl-D-glucoside | - |
| | | Color: light yellow | | | | N-Acetylglucosamine | + |
| | | Form: round | | | | Amygdalin | + |
| | | Elevation: convex | | | | Arbutin | + |
| | | Margin: entire | | | | Esculin | + |
| | | Surface form, etc.: smooth | | | | Salicin | + |
| | | Transparency: opaque | | | | Cellobiose | + |
| | | Viscosity: butyrous | | | | Maltose | + |
| Growth temperature test (°C) | | 10 | + | | | Lactose | + |
| | | 37 | + | | Fermentation test | Melibiose | + |
| | | 45 | + | | | Saccharose | + |
| Catalase reaction | | - | | | | Trehalose | + |
| | | | | | | Inulin | - |
| Oxidase reaction | | - | | | | Melezitose | + |
| O/F test (oxidation/fermentation) | | +/+ | | | | Raffinose | + |
| | | | | | | Starch | - |
| | Control | | | - | | Glycogen | - |
| | Glycerol | | | - | | Xylitol | - |
| | Erythritol | | | - | | Gentiobiose | + |
| | D-Arabinose | | | - | | D-Turanose | + |
| | L-Arabinose | | | + | | D-Lyxose | - |
| Fermentation test | Ribose | | | + | | D-Tagatose | - |
| | D-Xylose | | | - | | D-Fucose | - |
| | L-Xylose | | | - | | L-Fucose | - |
| | Adonitol | | | - | | D-Arabitol | - |
| | β-Methy-D-xyloside | | | - | | L-Arabitol | - |
| | Galactose | | | + | | Gluconate | + |
| | Glucose | | | + | | 2-Ketogluconate | - |
| | Fructose | | | + | | 5-Ketogluconate | - |

| | | | | | | | |
|---|---|---|---|---|---|---|---|
| +: positive, -: negative | | | | | | | |

As shown in the Examples below, strain SOK04BY has an excellent plant growth-promoting action. Therefore, by containing strain SOK04BY, the plant growth-promoting agent according to a preferable embodiment can significantly promote plant body growth. The plant growth-promoting agent of the preferable embodiment may consist of strain SOK04BY or may contain another component together with strain SOK04BY as long as the plant growth-promoting agent contains strain SOK04BY. Dead cells of strain SOK04BY may be used, but live cells thereof are preferably used to exert an excellent plant growth-promoting action.

The form of the plant growth-promoting agent is not particularly limited, and forms of a general microorganism material, such as granules, dust, wettable powder, a pack, granular wettable powder, microcapsules and an emulsion, are included. The plant growth-promoting agent can be prepared in any form and can be used according to the purpose. For example, the lactic acid bacterium can be adsorbed onto a pharmaceutically acceptable carrier and provided in the form of wettable powder, dust or granules. In this case, as the carrier, diatomaceous earth, clay, talc, pearlite, chaff, bone dust, white carbon or the like can be used. As a pharmaceutically acceptable additive, a surfactant, a dispersing agent, an auxiliary agent or the like can be used.

The concentration of the lactic acid bacterium (for example, strain SOK04BY) contained in the plant growth-promoting agent according to this embodiment is not particularly limited, and the concentration may be 1×10³ to 1×10¹¹ cfu (colony forming unit)/g, 1×10⁴ to 1×10¹¹ cfu/g or 1×10⁶ to 1×10¹⁰ cfu (colony forming unit)/g. The lactic acid bacterium may be a culture solution itself. For example, in the case of an agent used after being diluted before treating a seed, a plant body or a soil, the concentration of the lactic acid bacterium in the agent before dilution is preferably 1×10⁸ to 1×10¹¹ cfu/g. Also, in the case of a solution in which the bacterial strain is dispersed (for example, a solution obtained by diluting the agent), the concentration of the lactic acid bacterium at the time of the treatment of a seed, a plant body or a soil is preferably 1×10³ to 1×10¹⁰ cfu/ml, more preferably 1×10⁶ to 1×10⁸ cfu/ml.

In the plant growth-promoting method according to this embodiment, at least one selected from the group consisting of a seed, a plant body and a soil is treated with the lactic acid bacterium. The plant body here refers to a plant after germination and does not include a seed before germination. The plant body is preferably a growing plant body having a stem, a leave and a root, further preferably a plantlet. The plantlet refers to a young plant shortly after germination from a seed and is a plant body at or before transplanting time, such as a seedling. The soil may be a soil for growing a plant and includes the soil of a field, a potting soil, a nursery soil, a seeding soil and the like.

Examples of the method for treating such seed, plant body or soil are the following methods.

(1) A method in which a seed, a plant body and/or a soil is treated with a plant growth-promoting agent containing a liquid in which the lactic acid bacterium is dispersed (for example, a culture solution of a bacterial strain). This method includes: immersing the root part of a seedling before planting (transplanting) in the liquid in which the lactic acid bacterium is dispersed; spraying a leave, a stem or the like with the liquid in which the lactic acid bacterium is dispersed; irrigating a growing plant body and its rhizosphere with the liquid in which the lactic acid bacterium is dispersed (for example, irrigating a plantlet before transplanting and the soil in which the plantlet grows, irrigating a plant body after planting and the soil in which the plant body grows or the like); spraying or irrigating a soil before planting (for example, the soil of a field or a potting soil) with the liquid in which the lactic acid bacterium is dispersed; and spraying or irrigating a seeding soil or a nursery soil before seeding with the liquid in which the lactic acid bacterium is dispersed.
(2) A method in which a seed, a plant body and/or a soil is treated with a plant growth-promoting agent in the form of dust or granule prepared by powdering the lactic acid bacterium itself or adhering the lactic acid bacterium to a carrier. This method includes: dusting the growth-promoting agent in the form of dust or granule over the soil during raising a seedling; dusting over the soil of a field after planting; mixing in a potting soil or the soil of a field (blending); and mixing in a seeding soil, a nursery soil or the like before seeding (blending).

In this embodiment, examples of the plant to which the plant growth-promoting agent is applied include Solanaceae crops such as tomato, red pepper, eggplant, potato and petunia; Gramineae crops such as rice and corn; Liliaceae crops such as spring onion, onion, tulip and lily; Cucurbitaceae crops such as cucumber, watermelon and pumpkin; Brassicaceae crops such as cabbage, Chinese cabbage, daikon radish, stock, ornamental kale and potherb mustard; Chenopodiaceae crops such as spinach; Araceae crops such as taro, calla and pothos; Rosaceae crops such as strawberry, Japanese apricot, peach and apple; Leguminosae crops such as soybean and adzuki bean; Apiaceae crops such as carrot and parsley; Asteraceae crops such as burdock, lettuce, chrysanthemum, cosmos and sunflower; Iridaceae crops such as gladiolus; Plumbaginaceae crops such as statice; Gesneriaceae crops such as saintpaulia; Scrophulariaceae crops such as snapdragon and torenia; Caryophyllaceae crops such as carnation and gypsophila; Convolvulaceae crops such as morning glory; Amaryllidaceae crops such as narcissus; Orchidaceae crops such as cattleya and cymbidium; Ebenaceae crops such as persimmon; Moraceae crops such as fig; Vitaceae crops such as grape; Fagaceae crops such as chestnut; Rutaceae crops such as *Citrus unshiu* and lemon; Actinidiaceae crops such as kiwi and the like. The plant growth-promoting agent can be applied to at least any one kind of these plants. In an embodiment, the plant to which the plant growth-promoting agent is applied may be at least a kind selected from the group consisting of Solanaceae crops, Gramineae crops, Liliaceae crops and Cucurbitaceae crops.

According to this embodiment, by treating a seed, a plant body and/or a soil in which a plant grows using a bacterial strain having a plant growth-promoting action of the lactic acid bacteria, plant growth can be promoted, and the yields of vegetables, grains, fruits and the like can be increased.

### Examples

The invention is explained more specifically referring to Examples below, but the scope of the invention is not limited to these Examples.

### (Example 1: Influence on Growth·Yield of Tomato, Field Experiment (1))

Seeds of tomato (variety: Hausu Momotaro) were sowed (one seed per pot) in vinyl pots (diameter of 6 cm) filled with a commercial potting soil (Nippi Gardening Soil No. 1, manufactured by Nihon Hiryo Co., Ltd). Thirty-two days after seeding, the pots were irrigated with a 200-fold diluted solution of a lactic acid bacterium agent containing strain SOK04BY (a solution obtained by diluting 1 g of the lactic acid bacterium agent with 200 mL of distilled water) at 20 mL per pot, and this was used as a bacterium-treated section. As a control, pots were irrigated with distilled water at 20 mL per pot, and this was used as an untreated section. On the day after the irrigation, the seedlings of tomato were planted in a vinyl house. Specifically, the seedlings were planted at row width of 1.5 m and intrarow spacing of 0.5 m in four plants × three replications in each section. That is, in each section, three groups each including four plants were distributed in the house, and 12 plants in total were planted. The planting time was late April.

As the lactic acid bacterium agent, an agent prepared by thoroughly mixing and pulverizing 10 mass% of strain SOK04BY, 0.5 mass% of sodium lauryl sulfate, 4.5 mass% of sodium lignin sulfonate, 2.5 mass% of white carbon and 82.5 mass% of clay was used. The concentration of strain SOK04BY contained in the lactic acid bacterium agent was 1×10¹⁰ cfu/g.

Two weeks and four weeks after planting in the vinyl house, the growth of the tomato plants in the untreated section and the bacterium-treated section was examined. For the examination, the plant heights, the stem widths and the leaf positions (the numbers of true leaves) were measured two weeks after planting, and the plant heights, the stem widths, the leaf positions and the maximum leaf lengths were measured four weeks after planting. The average values of the respective sections were calculated, and the results are shown in Table 2 below. Because the tomato plants had compound leaves, each maximum leaf length was the length from the stem to the end of the leaf.

As shown in Table 2, the growth after planting was promoted significantly in the bacterium-treated section, in which the seedlings of tomato before transplanting (plantlets) were treated with strain SOK04BY, as compared to that in the untreated section.

**[Table 2]**

| Influence on Growth of Tomato Planted in Vinyl House | | | | | | | |
|---|---|---|---|---|---|---|---|
| Experimental Section | 2 Weeks After Planting | | | 4 Weeks After Planting | | | |
| | Plant Height (cm) | Stem Width (mm) | Leaf Position | Plant Height (cm) | Stem Width (mm) | Leaf Position | Maximum Leaf Length (cm) |
| Untreated Section | 29.3 | 5.6 | 7.5 | 59.6 | 15.9 | 12.6 | 42.3 |
| Bacterium-Treated Section | 32.3 | 8.2 | 8.3 | 68.8 | 18.6 | 13.4 | 54.5 |

Also, 75 days, 78 days, 83 days, 86 days and 89 days after planting, tomatoes were harvested, and the influence of strain SOK04BY on the yield of tomatoes was examined. The results are as shown in Table 3. The numbers of the harvested fruits in Table 3 are the yields of fruits to the third cluster. Also, the value in the brackets after the total number indicates the ratio of the total yield of the bacterium-treated section to the total yield of the untreated section which is regarded as 100. As shown in Table 3, the yield of tomatoes increased by 37% in the bacterium-treated section, which was treated with strain SOK04BY, as compared to that in the untreated section.

**[Table 3]**

| Influence on Yield of Tomatoes Planted in Vinyl House | | | | | | |
|---|---|---|---|---|---|---|
| Experimental Section | Number of Harvested Fruits (Fruits/12 Plants) | | | | | |
| | After 75 Days | After 78 Days | After 83 Days | After 86 Days | After 89 Days | Total |
| Untreated Section | 24 | 18 | 19 | 32 | 21 | 114 (100) |
| Bacterium-Treated Section | 42 | 29 | 27 | 36 | 22 | 156 (137) |

### (Example 2: Influence on Growth of Tomato, Pot Experiment)

Seedlings of tomato (variety: Hausu Momotaro) about 40 days after seeding were irrigated with a 200-fold diluted solution of the lactic acid bacterium agent containing strain SOK04BY at 10 mL per plant, and this was used as a bacterium-treated section. As a control, seedlings were irrigated with the same amount of distilled water, and this was used as an untreated section. On the day after the irrigation, the seedlings of tomato were transplanted to large pots (diameter of 20 cm) and cultivated in an unheated vinyl house. In each section, 20 pots were cultivated. The experiment included Experiment Example 1 in which the plants were cultivated in the vinyl house for 31 days from May to June and Experiment Example 2 in which the plants were cultivated in the vinyl house for 28 days from November to December. The plant weights were measured after the completion of cultivation in Experiment Example 1, and the plant weights and the plant heights were measured after the completion of cultivation in Experiment Example 2. The average plant weights and the average plant heights of the respective sections were determined, and the results are shown in Table 4 below.

As shown in Table 4, also in the pot experiment, the growth of tomato was promoted in the bacterium-treated section, which was treated with strain SOK04BY, as compared to that in the untreated section.

**[Table 4]**

| Influence on Growth of Tomato (Pot Experiment) | | | | | |
|---|---|---|---|---|---|
| | Experimental Section | Plant Wight (g/Plant) | Ratio of Plant Weight | Plant Height (cm) | Ratio of Plant Height |
| Experiment Example 1 | Untreated Section | 248.3 | 100 | - | - |
| | Bacterium-Treated Section | 280.0 | 113 | - | - |
| Experiment Example 2 | Untreated Section | 39.8 | 100 | 43.0 | 100 |
| | Bacterium-Treated Section | 43.5 | 109 | 44.2 | 103 |

### (Example 3: Influence on Yield of Tomatoes, Field Experiment (2))

The field experiment was conducted in the year following the field experiment of Example 1. Seedlings of tomato (variety: Hausu Momotaro) which had been grown in vinyl pots (diameter of 6 cm) were irrigated with a 200-fold diluted solution of the lactic acid bacterium agent containing strain SOK04BY at 20 mL per pot on the day before planting, and this was used as a bacterium-treated section. As a control, seedlings were irrigated with distilled water at 20 mL per pot, and this was used as an untreated section. In each section, the seedlings were planted in a vinyl house in 10 plants × three replications (30 plants in total) (at row width of 1.5 m and intrarow spacing of 0.5 m), and the tomato plants were cultivated. The planting time was in the middle of April.

The yields of tomatoes in the week after the harvest of tomatoes was started (0-1 week), in the next week (1-2 week) and in the following two weeks (2-4 weeks) were examined, and the results are shown in Table 5 below. Each yield of tomatoes in Table 5 is the mass of the tomato fruits harvested to the fifth cluster, and each value in the "Ratio to Untreated Section" is the ratio to the value of the untreated section which is regarded as 100.

As shown in Table 5, a significant effect of increasing the initial yield was obtained in the bacterium-treated section, which was treated with strain SOK04BY, as compared to the untreated section, and the total yield thereof was also increased.

**[Table 5]**

| Influence on Yield of Tomatoes (Field Experiment) | | | | |
|---|---|---|---|---|
| Experimental Section | Yield of Tomatoes in Each Period (g/30 Plants) | | | Total Yield |
| | 0-1 Week | 1-2 Week | 2-4 Weeks | (g/30 Plants) |
| Untreated Section | 14805 | 27338 | 51013 | 93155 |
| Bacterium-Treated Section | 18187 | 31704 | 51775 | 101666 |
| (Ratio to Untreated Section) | (123) | (116) | (101) | (109) |

### (Example 4: Influence on Yield of Rice, Field Experiment)

Seedlings of rice (variety: Koshihikari) which had been grown in nursery cabinets (internal size of 580x280x28 mm) for about 20 days after seeding were irrigated with a 200-fold diluted solution of the lactic acid bacterium agent containing strain SOK04BY at 1 L per nursery cabinet on the day before transplanting, and this was used as a bacterium-treated section. As a control, seedlings were irrigated with distilled water at 1 L per cabinet, and this was used as an untreated section. On the day after the irrigation, the seedlings of rice were transplanted to a paddy field, and the rice seedlings were cultivated. The transplanting time was in the middle of June. In the middle of October, crop estimate by unit acreage sampling was conducted, and the weights of brown rice per unit area were measured. The results of the weights of brown rice per 10 a (namely 1000 m²) paddy field are shown in Table 6 below.

As shown in Table 6, the growth of rice was promoted in the bacterium-treated section, which was treated with strain SOK04BY, as compared to that in the untreated section, and thus an increase in yield by about 8% was observed.

**[Table 6]**

| Influence on Yield of Rice | |
|---|---|
| Experimental Section | Weight of Brown Rice per Unit Area (kg/10 a) |
| Untreated Section | 515.2 |
| Bacterium-Treated Section | 557.3 |

### (Example 5: Influence on Growth of Rice, Pot Experiment)

In Experiment Example 1, seedlings of rice (variety: Hinohikari) about 20 days after seeding were irrigated with a 100-foled diluted solution of the lactic acid bacterium agent containing strain SOK04BY at 1 L per nursery cabinet, and this was used as a bacterium-treated section. As a control, seedlings were irrigated with the same amount of distilled water, and this was used as an untreated section. On the day after the irrigation, the seedlings of rice were transplanted to 500-mL cups at three plants per cup and cultivated for a month. In each section, six cups × four replications (24 cups in total) were cultivated.

In Experiment Example 2, seedlings of rice (variety: Koshihikari) about 20 days after seeding were used. During transplanting, the seedlings of rice were immersed in a cell suspension of strain SOK04BY (1×10⁸ cfu/g). Then, the seedlings were transplanted to 500-mL cups at three plants per cup and cultivated for a month, and this was used as a bacterium-treated section. As a control, seedlings which were immersed in distilled water instead of the cell suspension were used as an untreated section. In each section, six cups × four replications (24 cups in total) were cultivated.

The plant heights, the numbers of tillers, the weights of above-ground part and the root weights of rice after the one-month cultivation were examined. The average values of the respective sections were determined, and the results are shown in Table 7 below. As shown in Table 7, both in Experiment Example 1 and in Experiment Example 2, the number of tillers increased in the bacterium-treated section as compared to that in the untreated section. Considering that an ear of rice grows in one tiller, it is expected that the yield would increase when the number of tillers increases. Also, in Experiment Example 2, the weight of above-ground part of the bacterium-treated section was higher than that of the untreated section, and the growth was promoted also in view of this point. In Experiment Example 1, no difference in the weight of above-ground part was observed between the bacterium-treated section and the untreated section, but the root weight was higher in the bacterium-treated section than in the untreated section, and thus further growth could be expected.

**[Table 7]**

| Influence on Growth of Rice | | | | | |
|---|---|---|---|---|---|
| | Experimental Section | Plant Height (cm) | Number of Tillers | Weight of Above-Ground Part (g (Dry Weight)) | Root Weight (g (Dry Weight)) |
| Experiment Example 1 | Untreated Section | 54.1 | 9.38 | 1.27 | 0.30 |
| | Bacterium-Treated Section | 54.1 | 9.83 | 1.25 | 0.42 |
| Experiment Example 2 | Untreated Section | 84.6 | 5.88 | 3.04 | 0.74 |
| | Bacterium-Treated Section | 84.0 | 6.75 | 3.34 | 0.79 |

### (Example 6: Influence on Growth of Spring Onion, Pot Experiment)

Seeds of spring onion (variety: Kurosenbon) were sowed (three seeds per cell) in a 200-cell tray (volume per cell: 14 mL) filled with a commercial potting soil (Nippi Gardening Soil No. 1, manufactured by Nihon Hiryo Co., Ltd). Fifty days after seeding, the cells were irrigated with a 100-fold diluted solution of the lactic acid bacterium agent containing strain SOK04BY at 5 mL per cell, and this was used as a bacterium-treated section. As a control, cells were irrigated with distilled water at 5 mL per cell, and this was used as an untreated section. On the day after the irrigation, the seedlings of spring onion were transplanted to vinyl pots having a diameter of 10.5 cm at three plants per pot. In each section, the seedlings were transplanted in six pots × three replications (18 pots in total), and the growth was examined 90 days after transplanting. For the examination, the numbers per plant, the plant heights and the weights of above-ground part were measured, and the average values of the respective sections were calculated. The results are shown in Table 8 below.

As shown in Table 8, the growth of spring onion was promoted in the bacterium-treated section, which was treated with strain SOK04BY, as compared to that in the untreated section.

**[Table 8]**

| Experimental Section | Number | Plant Height (cm) | Weight of Above-Ground Part (g (Fresh Weight)) | Ratio of Weight of Above-Ground Part |
|---|---|---|---|---|
| Untreated Section | 2.9 | 55.2 | 16.87 | 100 |
| Bacterium-Treated Section | 3.0 | 59.7 | 23.79 | 141 |

### (Example 7: Influence on Growth of Red Pepper)

Ten grams of the lactic acid bacterium agent containing strain SOK04BY was blended with 2 L of a commercial nursery soil (Metro-Mix, manufactured by HYPONex Japan Corporation), and 100 cells of a 200-cell tray (volume per cell: 14 mL) were filled with the soil. Seeds of red pepper (variety: Hushimi Red Pepper) were sowed (one seed per cell) and irrigated with water at 5 mL per cell, and this was used as a bacterium-treated section. As a control, seeding and irrigation were conducted in the same manner as in the bacterium-treated section except that the lactic acid bacterium agent was not blended, and this was used as an untreated section. After the irrigation, the plants were cultivated in a greenhouse at 25°C, and a liquid fertilizer was applied 10 days after seeding. The plant heights and the weights of above-ground part were examined as the growth 22 days after seeding. The average plant heights and the average weights of above-ground part of the respective sections were determined. The experiment was conducted twice, and the results of each experiment are shown in Table 9 below.

In addition, in the first experiment, the plants were potted in vinyl pots having a diameter of 10.5 cm 22 days after seeding. The plants were potted in 12 pots × five replications (60 pots in total) in each section, and the plant heights, the numbers of leaves and the weights of above-ground part were examined as the growth 19 days after potting. Also in the second experiment, the plants were similarly potted 22 days after seeding, and the plant heights, the numbers of leaves and the weights of above-ground part were examined 15 days after potting. The results are shown in Table 10 below.

As shown in Tables 9 and 10, the growth of red pepper was promoted in the bacterium-treated section, which was treated with strain SOK04BY, as compared to that in the untreated section.

**[Table 9]**

| Influence on Growth of Red Pepper (22 Days After Seeding) | | | | | |
|---|---|---|---|---|---|
| | Experimental Section | Plant Height (cm) | Ratio of Plant Height | Weight of Above-Ground Part (g (Fresh Weight)) | Ratio of Weight of Above-Ground Part |
| First Experiment | Untreated Section | 6.03 | 100 | 0.14 | 100 |
| | Bacterium-Treated Section | 7.94 | 132 | 0.27 | 193 |
| Second Experiment | Untreated Section | 7.63 | 100 | 0.54 | 100 |
| | Bacterium-Treated Section | 10.00 | 131 | 0.86 | 160 |

**[Table 10]**

| Influence on Growth of Red Pepper (After Potting) | | | | | |
|---|---|---|---|---|---|
| | Experimental Section | Plant Height (cm) | Number of Leaves | Weight of Above-Ground Part (g (Fresh Weight)) | Ratio of Weight of Above-Ground Part |
| First Experiment (After 19 Days) | Untreated Section | 17.2 | 8.5 | 3.69 | 100 |
| | Bacterium-Treated Section | 21.6 | 9.8 | 4.90 | 133 |
| Second Experiment (After 15 Days) | Untreated Section | 16.0 | 9.7 | 4.82 | 100 |
| | Bacterium-Treated Section | 19.4 | 11.8 | 6.17 | 128 |

### (Example 8: Influence on Growth of Cucumber)

By blending 0.1 L of a commercial nursery soil (Metro-Mix, manufactured by HYPONex Japan Corporation) and blending 1 L of the soil per 1 g of the lactic acid bacterium agent containing strain SOK04BY, a 100-fold blended soil and a 1000-fold blended soil were prepared, respectively. Sixteen cells of a 128-cell tray (volume per cell: 22 mL) were filled with the blended soils (each in three replications), and seeds of cucumber (variety: Zubari 163) were sowed (one seed per cell). The cells were irrigated with water at 7 mL per cell, and they were used as bacterium-treated sections (a 100-fold blended section and a 1000-fold blended section). As a control, seeding and irrigation were conducted in the same manner as in the bacterium-treated sections except that the lactic acid bacterium agent was not blended, and this was used as an untreated section. After the irrigation, the plants were cultivated in a greenhouse in which the minimum temperature was kept at 20°C. The experiment was conducted twice, and the plant heights and the weights of above-ground part were examined as the growth, 11 days after seeding in the first experiment, and 10 days after seeding in the second experiment. The average plant heights and the average weights of above-ground part of the respective sections were determined. The results of each experiment are shown in Table 11 below.

In addition, in the first experiment, the plants of the untreated section and the 100-fold blended section were potted in vinyl pots having a diameter of 10.5 cm 11 days after seeding. The plants were potted in six pots × three replications (18 pots in total) in each section, and the plant heights and the weights of above-ground part were examined as the growth 13 days after potting. Also in the second experiment, the plants were similarly potted 10 days after seeding, and the plant heights and the weights of above-ground part were examined nine days after potting. The results are shown in Table 12 below.

As shown in Tables 11 and 12, the growth of cucumber was promoted in the bacterium-treated sections, which were treated with strain SOK04BY, as compared to that in the untreated section.

**[Table 11]**

| Influence on Growth of Cucumber (After Seeding) | | | | |
|---|---|---|---|---|
| | Experimental Section | Plant Height (cm) | Weight of Above-Ground Part (g (Fresh Weight)) | Ratio of Weight of Above-Ground Part |
| First Experiment (After 11 Days) | Untreated Section | 5.0 | 0.49 | 100 |
| | 100-fold Blended Section | 6.7 | 0.71 | 145 |
| | 1000-fold Blended Section | 5.4 | 0.51 | 104 |
| Second Experiment (After 10 Days) | Untreated Section | 7.4 | 0.67 | 100 |
| | 100-fold Blended Section | 9.2 | 0.96 | 143 |
| | 1000-fold Blended Section | 7.7 | 0.74 | 111 |

**[Table 12]**

| Influence on Growth of Cucumber (After Potting) | | | | |
|---|---|---|---|---|
| | Experimental Section | Plant Height (cm) | Weight of Above-Ground Part (g (Fresh Weight)) | Ratio of Weight of Above-Ground Part |
| First Experiment (After 13 Days) | Untreated Section | 23.0 | 8.34 | 100 |
| | 100-fold Blended Section | 28.4 | 11.46 | 137 |
| Second Experiment (After 9 Days) | Untreated Section | 12.5 | 3.97 | 100 |
| | 100-fold Blended Section | 16.3 | 5.79 | 146 |

### Industrial Applicability

This embodiment can contribute to efficient farming because this embodiment can promote plant body growth and increase the yields of vegetables, grains, fruits and the like.

## Claims

1. A plant growth-promoting agent containing a lactic acid bacterium having a plant growth-promoting action.

2. The plant growth-promoting agent according to claim 1, wherein the lactic acid bacterium having a plant growth-promoting action is a microorganism belonging to *Lactobacillus.*

3. The plant growth-promoting agent according to claim 2, wherein the lactic acid bacterium having a plant growth-promoting action is a microorganism classified as *Lactobacillus plantarum.*

4. The plant growth-promoting agent according to claim 3, wherein the lactic acid bacterium having a plant growth-promoting action is *Lactobacillus plantarum* strain FERM BP-21501.

5. The plant growth-promoting agent according to any one of claims 1 to 4 which is used for treating at least one selected from the group consisting of a seed, a plant body and a soil.

6. The plant growth-promoting agent according to any one of claims 1 to 4 which is used for treating a plantlet.

7. A plant growth-promoting method **characterized by** treating at least one selected from the group consisting of a seed, a plant body and a soil with a lactic acid bacterium having a plant growth-promoting action.

8. The plant growth-promoting method according to claim 7, wherein the lactic acid bacterium having a plant growth-promoting action is *Lactobacillus plantarum* strain FERM BP-21501.

9. The plant growth-promoting method according to claim 7 or 8, wherein the plant body that is treated is a plantlet.
